# EUROPEAN PATENT APPLICATION

(11) **EP 4 289 350 A1**
(43) Date of publication of application: **13.12.2023**
(21) Application number: 22178087.7
(22) Date of filing: 09.06.2022
(51) Int. Cl.: A61B 5/055, A61B 5/00, G01R 33/34

(54) **RADIO FREQUENCY HEAD COIL WITH AN AUDIO REPRODUCTION SYSTEM**

(71) Applicant: Koninklijke Philips N.V., 5656 AG Eindhoven (NL)
(72) Inventor: VOGTMEIER, Gereon, Eindhoven (NL); WEISS, Steffen, Eindhoven (NL); LEUSSLER, Steffen, 5656AG Eindhoven (NL)
(74) Representative: Philips Intellectual Property & Standards

(57) **Abstract**

An RF head coil for acquisition of magnetic resonance signals from a patient's head according to the invention comprising an examination volume to receive the patient's head. Receiver elements are located around the examination volume and configured to provide a lateral access into the examination volume. An audio reproduction system with one or more audio outputs is integrated in the RF head coil. One or more flexible inserts fit between the integrated audio output(s) and the patient's ear position. When the patient's head to be examined is positioned in the RF head coil, the flexible inserts may be positioned along the lateral direction between the integrated audio outputs and the patient's outer ear.

## Description

### FIELD OF THE INVENTION

The invention pertains to a radio frequency (RF) head coil with an audio reproduction system.

### BACKGROUND OF THE INVENTION

Such a radio frequency (RF) head coil is known from the US-patent application US2015/141803.

The known head coil comprises a fixed coil casing with adjustable cushioned head-restraining ear pieces including an audio-delivery system mounted in the head coil in recesses of the RF coil. The ear pieces are mechanically displaceable by way of an assembly of rods and ball-and-socket joints.

### SUMMARY OF THE INVENTION

An object of the invention is to provide an RF head coil with an integrated audio reproduction system that is simpler to position and has a good audio quality to the listener.

This object is achieved by an RF head coil for acquisition of magnetic resonance signals from a patient's head according to the invention comprising
- an examination volume to receive the patient's head,
- receiver elements located around the examination volume and configured to provide a lateral access into the examination volume,
- an audio reproduction system with one or more audio outputs integrated in the RF head coil and
- one or more flexible inserts that fit between the integrated audio output(s) and the patient's ear position.

The RF head coil of the invention is provided with an integrated audio reproduction system. The audio reproduction system supports audio communication between the patient to be examined in an magnetic resonance examination system and operating staff outside the magnetic resonance examination system's magnet or even outside the examination room. That is, the audio reproduction system has similar functionality as a conventional audio head set that the patient to be examined may where during imaging in the magnetic resonance examination system. According to the invention the audio reproduction system has audio output(s) and optionally also supporting electronics, connections for power and data communication leads and its structural components to hold the audio output integrated in the RF head coil. The integrated configuration of the RF head coil and the audio reproducrtion system does not involve a large number of additional haredware components and is easy to manufacture .For example, the audio output may be a speaker integrated in the structure of the RF head coil. In another example air tubing may be provided to the audio output in the RF head coil. The air tubing may be combined with the external cabling of the RF head coil. Thus, the RF head coil of the invention having the audio reproduction system integrated in the RF head coil achieves that there is no need for the patient to be examined to wear a generally uncomfortable in-ear head set with separate power and data communication leads that take space in the examination zone of the RF head coil. The RF head coil has a lateral access, i.e. transverse to the longitudinal axis of the patient's body and along the anatomical anterior-posterior (AP) direction when the patient's head is in position in the RF head coil. Hence, when the patient's head to be examined is positioned in the RF head coil, the flexible inserts may be easily positioned along the lateral direction between the integrated audio outputs and the patient's outer ear. The inserts may be of a flexible material so that they fit snuggly between the patient's outer-ear and the integrated audio outputs. There may be provided several sizes of the flexible inserts that may fit according to the patient's head size. The flexible inserts may be configured to comfortably fit 'over ear' and ensure good acoustic and sound tight transmission between the integrated speakers and the patient's ears.

The easy insertion of the flexible inserts relies on the lateral access into the RF head coil's examination zone with the patient's head in place. The prior art as disclosed in the US-patent application US2015/141803 has not recognised this option. The known head coil is provided with mechanical manipulators to position its earpieces mounted to the frame of the RF coil. Further, conventional bird-cage type head coils typically have only longitudinal access in that the birdcage type head coil and the patient's head are positioned by relative translation along the patient's longitudinal axis. When the patient's head is properly positioned in the conventional bird-cage type RF coil's examination zone, there is hardly room for access towards the patient's ears. Therefore, it is customary in the prior art to employ conventional in-ear audio headsets with separate power and data leads in combination with conventional bird-cage type RF head coils.

These and other aspects of the invention will be further elaborated with reference to the embodiments defined in the dependent Claims.

A practical example of the RF head coil of the invention further comprises a base member and a cover member, the receiver elements being mounted to the base member and/or the cover member detachable from or partly displaceable from one another between a closed an open state, such that in the open state the lateral access is transersely towards the base member and in the closed state the receiver elements are located around the examination volume. In operation the cover member may be removed or pivoted with respect to the base member. This allows lateral access to the base member and the patient's head may be placed onto the base member. The base member has the audio outputs mounted near the position of the patient's ears. While the cover member is removed or pivoted away, lateral access to the patient's head in the base member is easy to place the flexible inserts to provide comfort and good acoustic transmission from the speakers in the base member to the patient's ears.

The flexible inserts may be provided as disposables or made to be easy cleanable or even desinfectable for multiple use. The flexible inserts may be made by three-dimensional (3D) printing techniques of flexible materials that may be biocompatible and the stiffness of the material may be adapted by the 3D-printing process. The flexible inserts may have a rigid interface to fit to the audio output mounted in the RF head coil and a flexible side facing, when in use, to the patient's ear. By 3D-printing good correspondence with the patient's head size and shape of the inserts is obtained.

The RF head coil comprises a set of receiver elements, e.g., in the form of electrically conducting coil loops that may be tuned to resonance to pick up magnetic flux due to magnetic resonance signals. According to the invention an acoustic resonator is provided in the base member and/or the cover member near or around the examination zone. These acoustic resonators may be provided between these receiver elements. The acoustic resonator is configured to amplify the sound level ant the audio outputs mounted in the structure of the RF head coil. To control the acoustic behaviour of the RF head coil acoustic damping material be disposed near the positions of the patient's ears.

The audio outputs may be provided with actuators to generate sound waves. These actuators may be piezo-electronically driven. To that end the audio outputs are formed by piezo-electrical speakers. Further, the RF head coil may be provided with one or more electronic pre-amplifiers coupled with the receiver elements to pre-amplify the voltage signal due to the picked up magnetic flux. One or several of these electronic pre-amplifiers may be connected to the actuators of the audio outputs, e.g. formed by the piezoelectric speakers to power these speakers by the electronic pre-amplifier's output. The piezo-electrically driven actuators can be operated with high electric-impedance wiring to enhance RF safety. To employ the acoustic resonator body is particularly advantageous in combination with the piezo-electrically driven actuators. In general piezo-electrical actuators have a relatively low frequency response especially at low acoustic frequencies. This may be compensated by the resonant design of the acoustic resonator bodies provided in the RF head coil.

The RF head coil of the invention may be a flexible head coil in which the receiver elements are arranged in a flexible manner with respect to each other and the piezo-electronic actuators and the pre-amplifiers are combined on one or several common pcb-boards. This embodiment provides for a flexible RF coil with an integrated audio output. The common pcb-boards that hold the electronic pre-amplifiers for the magnetic resonance signals can also be employed to mount the electronics to drive the piezo-electric actuators of the acoustic output. In this way there is no need for speakers components to mount the electronics for the audio output.

The invention also pertains to a workflow method for positioning a patient's head to be examined into the examination zone of a radio frequency head coil of the invention. The workflow steps include to move the patient head first along the lateral axis until the head is in position in the examination zone and subsequently insert the flexible inserts in between the position of the patient's ears and the respective audio outputs. That is, the workflow steps to move the patient head first along the lateral axis until the head is in position in the examination zone while the cover member is detached form the cover member and when the flexible inserts are positioned in between the position of the patient's ears and the respective audio outputs finally place the cover member over the examination zone onto the base member. Because the patient's head and subsequently the flexible inserts are positioned in the examination zone and between the patient's ears and the audio outputs that are integrated in the RF head coil, respectively along the lateral access, access for the patient as well as for support staff to position the flexible inserts is easy. This is in particular the case when an RF head coil is employed that comprises a base member and a cover member that are displaceable relative to one another. With the cover member removed, rotated away or displaced, the examination zone is laterally accessible without structural components obstructing access in particular to the region between the patient's ears and audio outputs in the RF coil, e.g. in the base member. Thus, the workflow involves to, while the cover member is removed, rotated away or displaced, from the cover member, move the patient head first along the lateral axis until the head is in position in the examination zone and when the flexible inserts are properly positioned finally place the cover member over the examination zone onto the base member.

Especially, access to place the flexible inserts is significantly easier for an RF head coil according to the invention having a lateral access as compared to for a conventional RF birdcage head coil that can only be accessed longitudinally and when the patient's head is in position, the region near the patient's rears is virtually inaccessible for the access at the RF birdcage coil's longitudinal end. The flexible inserts may be positioned while the patient's head is laterally accessible and the flexible inserts may be shifted along the AP-direction and along the longitudinal (feet-head (FH)) direction to accurately position them in position to achieve an acoustic path between the audio output (microphone) and the patient's ears.

These and other aspects of the invention will be elucidated with reference to the embodiments described hereinafter and with reference to the accompanying drawing wherein

### BRIEF DESCRIPTION OF THE DRAWINGS

Fig. 1 shows an outline of an RF head coil in which the invention is employed and
Fig. 2 shows a diagrammatic cross section of the RF head coil of Fig. 1 and
Fig. 3 shows a flow chart representing an implementation of the workflow of the invention.

### DETAILED DESCRIPTION OF THE EMBODIMENTS

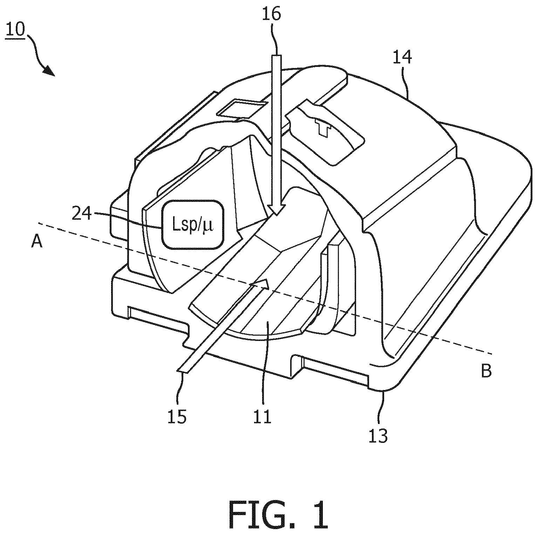

Fig. 1 shows an outline of an RF head coil 10 in which the invention is employed. The examples shows a 32-channel head coil that includes a base member 13 and a cover member 14 in which the receiver elements in the form of coil conductor elements 12 are mounted. When the cover member 14 is placed over the base member 13 the coil conductor elements 12 in the base and cover member respectively are electrically coupled so as to form a resonant structure around the examination zone 11 that is able to generate a *B₁*⁺-RF excitation field in transmit mode and in receive mode to pick up flux associated with magnetic resonance signals that are generated in the head of the patient to be examined. When the cover member is removed or displaced, the examination zone is freely laterally 16 (i.e. transverse to the RF head coil's longitudinal axis 15) accessible to place the patient's head over the base member. When in use in a magnetic resonance examination system, the longitudinal axis 15 may be along the orientation of the magnetic resonance examination system's main magnetic field and the lateral access may be transverse to a patient support's surface for example vertically. The audio reproduction system is (at least in part) integrated in the base member and the audio output, 24, e.g. a loudspeaker that may be combined with a microphone is visible.

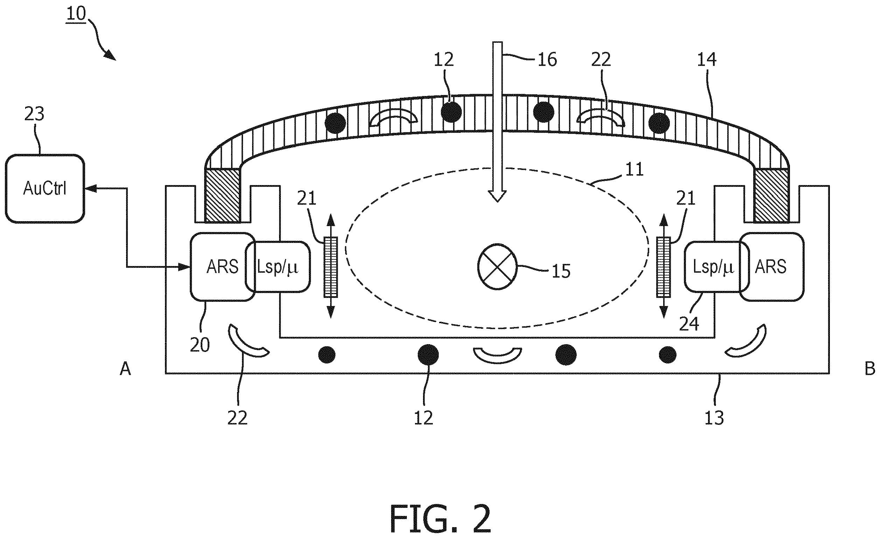

Fig. 2 shows a diagrammatic cross section along the line A-B and in more detail of the RF head coil of Fig. 1. The cover member 14 is detachable mounted in the base member 3. The electrical conductors 12 forming the receiver elements of the RF head coil 10 that are part of the resonant structure of the RF head coil are provided in both the cover member and the base member. The mechanical connection between the cover member 13 and the base member 14 contains also electrical contacts so that in its closed state the receiver elements of the RF head coil form a resonant structure in the Larmor frequency band. Alternatively to the detachable cover member 14, the cover member 14 may be coupled to the base member by way of a pivot, so that the cover member may be rotated away to allow access along the lateral access 16. The audio reproduction system 20 is integrated in the base member 13. The audio receiver system 20 is configured to communicate with an audio controller 23 that may be positioned outside of the RF head coil, e.g. as part of the magnetic resonance examination system control system. The audio reproduction system 20 is provided with audio outputs, e.g. loudspeakers that may also be combined with a microphone. The audio reproduction system enables audio communication between radiology staff with the patient to be examined and also provides for audio e.g. in form of music, to the patient to be examined during the examination procedure. Flexible inserts 21 are provided that may be inserted between the audio output 24 and the position of the patient's ears to ensure a high quality and comfortable acoustic path between the audio output 24 and the patient's auditory system. The flexible inserts 21 can be reached from the lateral direction to manipulated them along the AP-direction 16 and along the FH direction 15 to place them properly with respect to the audio output 24 and the patient's ears. These inserts 21 accurate the comfortable and good quality audio coupling, for differently sized heads of patients. Further, in the base member 13 and the cover member 14 the resonator elements 22 are provided, e.g. shaped as acoustic resonant cavities integrated between the receiver elements in the structure of the base and cover members 13,14. These acoustic resonant cavities serve to resonantly amplify the sound level and the patient's ears .

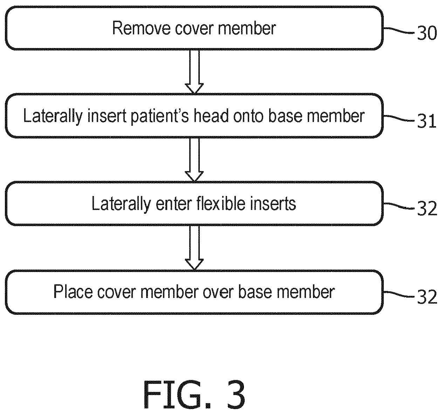

Fig. 3 shows a flow chart representing an implementation of the workflow of the invention. In the initial step 30 the cover member is removed, e.g. detached or pivoted away from the base member. This renders, the base member is laterally 16 accessible. Then the patient is position with the patient's head onto the base member by in step 31 moving the patient's head laterally in position with the patient's ears near the audio outputs. Then the flexible inserts 21 are laterally entered in step 32 into the space between the audio output 24 and the patient's ears and may be moved laterally and/or longitudinally so as to achieve the comfortable good quality audio coupling to the patient's ears. That is, from the lateral direction the flexible inserts may be positioned by moving them along the AP-direction 16 and the FH-direction 15 while the patient's head is position on the base member 12. In step 33 the cover member 14 is placed on top of the base member 13, ensuring mechanical and electrical connections between the receiver elements of the base and cover members. Then the image acquisition procedure can commence. After image acquisition is completed the cover member may be removed, the flexible inserts taken out and the patient removed laterally from the base member.

## Claims

1. A radio frequency (RF) head coil for acquisition of magnetic resonance signals from a patient's head comprising
- an examination volume to receive the patient's head,
- receiver elements located around the examination volume and configured to provide a lateral access into the examination volume,
- an audio reproduction system with one or more audio outputs integrated in the RF head coil and
- one or more flexible inserts that fit between the integrated audio output(s) and the patient's ear position.

2. A radio frequency head coil as claimed in Claim 1, further comprising
- a base member and a cover member,
- the receiver elements being mounted to the base member and/or the cover member
- detachable from or partly displaceable from one another between a closed and an open state, such that in the open state the lateral access is towards the base member and in the closed state the receiver elements are located around the examination volume.

3. A radio frequency head coil as claimed in Claim 1 or 2, wherein the inserts are made by a 3D printing process on the basis of prior knowledge of patient's head shape.

4. A radio frequency head coil as claimed in any one of Claims 1 to 3, wherein an acoustic resonator body is located near the examination volume.

5. A radio frequency head coil as claimed in any one of Claim 1 to 4, wherein the audio output has a piezo electrical driven actuator to generate sound waves.

6. A radio frequency head coil as claimed in any one of Claims 1 to 5, wherein electronic preamplifiers are mounted with the receiver elements for pre-amplification of the magnetic resonance signals and the electronic pre-amplifiers are further configured to amplify input signals to the integrated audio output(s).

7. A radio frequency head coil as claimed in Claim 5 or 6, wherein the receiver elements are arranged in a flexible manner with respect to each other and the piezo-electronic actuators and the preamplifiers are combined on one or several common pcb-boards.

8. A workflow method for positioning a patient's head to be examined into the examination zone of a radio frequency head coil of any one of Claims 1 to 7, the method comprising the workflow steps to
- while the cover member is detached form the cover member, move the patient head first along the lateral axis until the head is in position in the examination zone
- subsequently insert the flexible inserts in between the position of the patient's ears and the respective audio outputs
- finally place the cover member over the examination zone onto the base member.

9. A workflow method for positioning a patient's head to be examined into the examination zone of a radio frequency head coil as claimed in Claim 8, the method comprising the workflow steps to move the patient head first along the lateral axis until the head is in position in the examination zone while the cover member is detached form the cover member and
when the flexible inserts are positioned in between the position of the patient's ears and the respective audio outputs finally place the cover member over the examination zone onto the base member.
